# EUROPEAN PATENT APPLICATION

(11) **EP 1 810 667 A1**
(43) Date of publication of application: **25.07.2007**
(21) Application number: 06001232.5
(22) Date of filing: 20.01.2006
(51) Int. Cl.: A61K 9/14, A61K 9/16, A61K 9/20, A61K 31/40

(54) **Pharmaceutical composition comprising amorphous atorvastatin**

(71) Applicant: KRKA, tovarna zdravil, d.d., Novo mesto, 8501 Novo mesto (SI)
(72) Inventor: Kroselj, Vesna, 8310 Sentjernej (SI); Jakse, Renata, 8220 Smarjeske Toplice (SI); Stimac, Anton, 1000 Ljubljana (SI); Pisek, Robert, 1000 Ljubljana (SI); Gartner, Andrej, 1000 Ljubljana (SI); Ocepek, Uros, 8000 Novo mesto (SI)
(74) Representative: UEXKÜLL & STOLBERG

(57) **Abstract**

The invention relates to a pharmaceutical composition of atorvastatin of improved stability and bioavailability which comprises small amounts of an alkali metal additive and which is exposed to an atmosphere comprising 1 to 16 % by volume of oxygen.

## Description

### Field of the invention

The invention relates to a pharmaceutical composition of amorphous atorvastatin which has an improved stability and bioavailability as well as a process for its preparation.

### Background of the invention

Atorvastatin is a member of the class of drugs called statins. Statins suppress cholesterol biosynthesis by competitively inhibiting 3-hydroxy-3-methyl-glutaryl-coenzyme A reductase which catalyzes the conversion of HMG-CoA to mevalonate, which is the rate determining step in the biosynthesis of cholesterol. They are currently the most therapeutically effective drugs available for the treatment of hyperlipidemia and hypercholesterolemia, both of which are risk factors for arteriosclerosis and coronary heart disease.

Atorvastatin, [R-(R*, R*)]-2-(4-fluorophenyl)-β, δ-dihydroxy-5-(1-methylethyl)-3-phenyl-4-[(phenylamino)carbonyl]-1H-pyrrole-1-heptanoic acid, its lactone form and its calcium salt are known in the art, and processes for the preparation of atorvastatin and its key intermediates are disclosed in, for example, the United States Patents 5,003,080; 5,097,045; 5,103,024; 5,124,482; 5,149,837; 5,155,251; 5,216,174; 5,245,047; 5,248,793; 5,280,126; 5,342,952, 5,397,792; 4,681,893; 5,273,995, and 5,298,627.

Atorvastatin calcium can exist in an amorphous form or in different crystalline forms which are disclosed in the patent applications WO 97/3958, WO 97/3959, WO 01/36384, WO 02/41834, WO 02/43732, WO 02/51804, and WO 02/57229. The processes for the preparation of amorphous atorvastatin calcium are described in the patent applications WO 97/3960, WO 00/71116, WO 01/28999, WO 01/42209, WO 02/57228, and WO 02/59087.

According to the prior art processes, crystalline and amorphous forms of atorvastatin calcium are used for the preparation of solid oral dosage forms.

Already in EP 680 320 it is pointed out that atorvastatin has an insufficient stability. It is stated that atorvastatin is susceptible to heat, humidity, low pH and light, particularly UV radiation. As basic stabilizers calcium, magnesium, and lithium salts, e.g. hydroxides, oxides or carbonates, are proposed.

Various further attempts have been made to stabilize atorvastatin. WO 00/35425 discloses attempts to stabilize statin formulations using buffering agents capable of providing a pH in the range from 7 to 11.

US patents 5,686,104 and 6,126,971 disclose oral pharmaceutical formulations of atorvastatin in which the formulations are described as being stabilized by the addition of a pharmaceutically acceptable alkaline earth metal salt. According to these patents, large amounts of alkaline earth metal salt are required to stabilize the formulation.

WO 01/93859 solves the problem of stabilizing atorvastatin by using a substance which is capable of binding and neutralizing carbon dioxide. It is pointed out that gastric troubles may be caused if a medicine with a high content of alkaline substances is administered to patients.

WO 2004/032920 describes a stabilized pharmaceutical preparation comprising amorphous atorvastatin calcium and magnesium oxide which is exposed to an inert gas atmosphere of equal to or less than 2.4% oxygen content.

A similar type of stabilization of amorphous atorvastatin calcium is disclosed in WO 2005/011638 wherein atorvastatin alone or in a mixture with other solid substances is exposed to a partial pressure of oxygen of only 2 kPa or less. The corresponding very low oxygen partial pressure is achieved either by use of oxygen absorbers or a specific packaging process using inert gas, which are both measures complicating the manufacturing process.

However, the compositions according to the prior art still suffer from the problems that they either require a very substantial reduction of the oxygen content of the surrounding atmosphere, or need a specific packaging process or use basic stabilizers such as calcium carbonate and magnesium oxide, often in large amounts, which stabilizers virtually insoluble and therefore difficult to be processed to give a composition wherein they evenly surround the active ingredient.

It is therefore an object of the present invention to provide a composition avoiding these drawbacks and which is not only highly stable, but also provides a high bioavailability of the active ingredient.

This object is surprisingly solved by the composition according to claims 1 to 18. The invention also relates to the process according to claims 19 to 22.

### Detailed description of the invention

The pharmaceutical composition according to the invention comprises amorphous atorvastatin, a salt or ester thereof as active ingredient and an alkali metal additive selected from sodium hydroxide and potassium hydroxide and is exposed to an atmosphere comprising 1 to 16 % by volume of oxygen.

It has proved advantageous that the composition is exposed to an atmosphere comprising 2 to 12 % by volume of oxygen, and in particular 4 to 10 % by volume of oxygen.

Further, it is to be understood that the term amorphous includes amorphous and partly crystallized forms.

Useful salts of atorvastatin are the calcium, magnesium, sodium salt, and potassium salts. The active ingredient is preferably amorphous atorvastatin calcium.

The composition preferably comprises sodium hydroxide as alkali metal additive. The composition usually comprises 0.01 to 5, preferably 0.1 to 3 and most preferred 0.2 to 1 % by weight of the alkali metal additive.

The atmosphere with the required reduced oxygen content or reduced oxygen partial pressure may be obtained by the use of a reduced pressure atmosphere, e.g. by creating a partial vacuum by means of a suitable pump or by partial freezing or liquefying the atmosphere, or by the use of an inert gas, such as nitrogen or argon, or by the use of oxygen absorbents, even though the high efficacy of such absorbents leading to very low oxygen levels is normally not necessary.

It is preferred that the atmosphere comprises 2 to 12 % by volume of oxygen, and in particular 4 to 10 % by volume of oxygen.

It has surprisingly been found out that the amorphous atorvastatin compositions according to the invention are not only showing a high stability, even if only a small amount of the specific alkali metal salt additive, together with only a slightly reduced oxygen partial pressure is used, but that they have at the same time an increased bioavailability.

It has surprisingly also been shown to be beneficial for the stability and bioavailability of the composition to include specific disintegrants. Therefore, a composition is preferred which comprises at least one of croscarmelose sodium and crospovidone as disintegrant. Particularly preferred is a composition which comprises a mixture of corscarmelose sodium and crospovidone. This mixture is usually included in the composition in an amount of 5 to 15 and preferably 8 to 12 and most preferred about 10 % by weight.

Further, the composition normally comprises 2 to 20, preferably 5 to 15 and most preferred 8 to 13 % by weight of the active ingredient.

The composition can also comprise at least one of several other components, such as diluents or carriers, surface active agents, antioxidants and chelating agents, further disintegrants, binders, lubricants and glidants.

Suitable diluents or carriers include pharmaceutically acceptable fillers such as lactose, microcrystalline cellulose, silicified microcrystalline cellulose, powdered cellulose, calcium carbonate, dibasic calcium phosphate, tribasic calcium phosphate, sucrose, glucose, dextrates, dextrins, dextrose, fructose, lactitol, mannitol, sorbitol, starch and/or mixtures of the foregoing. The most preferred diluents or carriers are microcrystalline cellulose and lactose as well as hydrates of lactose.

Suitable surface active agents include emulsifying agents such as those commonly known to one skilled in the art. Surface active agents include, but are not limited to sodium laurylsulfate, glyceryl esters, polyoxyethylene glycol esters, polyoxyethylene glycol ethers, polyoxyethylene sorbitan fatty acid esters, sulphate containing surfactants, or polyoxyethylene/polyoxypropylene copolymers. The most preferred is sodium laurylsulfate.

Possible antioxidants include, but are not limited to, vitamin E acetate, α-tocopherol, ascorbyl palmitate, butylated hydroxyanisole (BHA), butylated hydroxytoluene (BHT), propyl gallate, dithiotreitol, or tocopherol polyethyleneglycol succinate (TPGS). Chelating agents can also be used as antioxidants, for example EDTA or cyclodextrins.

Suitable further disintegrants, i.e. apart form the croscarmellose sodium and crospovidone mentioned above, are sodium starch glycolate, corn starch, potato starch, maize starch and modified starches, calcium silicates, low substituted hydroxypropylcellulose and the like.

Suitable binders are selected from polyvinylpyrolidone, starch grades (pre-gelatinized or plain), cellulose derivatives such as hydroxypropylmethyl cellulose (HPMC), hydroxypropyl cellulose (HPC) and carboxymethyl cellulose (CMC) and their salts and gelatine, the most preferred being HPC.

Lubricants are preferably selected from the group consisting of magnesium stearate, magnesium lauryl sulfate and sodium stearyl fumarate, sucrose esters of fatty acids, polyethylene glycol and stearic acid.

Glidants are preferably selected from the group consisting of silicon dioxide, talc and aluminium silicate.

Possible are also sweeteners, flavourings, colouring agents, or opacifying agents and pigments.

The compositions according to the present invention may be in any form such as, for example, tablets, orally dispersible pharmaceutical formulations, capsules, pellets, and granulates, which are suitable to be stored in a gas exchange non-permeable packaging Preferably, the compositions are in form of a tablet, and in particular a tablet having a coating. The coating can be applied by methods known in the art. As coating agents, polymers can be used, such as cellulose derivatives, like hydroxypropylmethyl cellulose, hydroxypropyl cellulose, methyl cellulose, and sodium carboxymethylcellulose, povidone, polyvinyl alcohol, methacrylic acid copolymers, and methacrylic esters copolymers.

It has surprisingly been found out that specific coatings are able to substantially contribute to the stabilization of the tablets according to the invention. In this connection waxy materials which are surface active are particularly useful. Preferably they have a melting point of about 33°C to about 64°C and in particular about 35°C to about 55°C. They usually have a HLB value of from about 1 to about 14, in particular from about 7 to about 14.

A preferred waxy and surface active material comprises fatty acid esters of glycerol (in particular C₁₆ to C₁₈ fatty acid esters of glycerol), PEG esters and/or free PEG (lauryl macrogol glycerides).

The waxy and surface active material can be applied alone or in combination with other coatings, in particular those based on carboxymethylcellulose sodium (NaCMC) or polyvinyl alcohol (PVA).

The pharmaceutical composition according to the invention may include one or more of a 10 mg atorvastatin dosage unit, a 20 mg atorvastatin dosage unit, a 40 mg atorvastatin dosage unit, and an 80 mg atorvastatin dosage unit. Further, the composition is preferably used in the treatment of hypercholesterolemia and hyperlipidemia.

It is further preferred that the composition is present in a packaging, with a blister packaging or a bottle being preferred. Thus a packaged composition is formed. The packaging can be provided with means for trapping and disposal of free oxygen. Moreover, the composition is preferably enclosed in a substantially gas exchange non-permeable material as packaging which has an atmosphere with the required reduced oxygen content. The substantially gas exchange non-permeable packaging is preferably selected from the group consisting of an A1/A1 blister package, an Al-polychloro-3-fluoroethylene homopolymer/PVC laminate blister or a bottle.

The compositions according to the invention may be prepared by well known processes, such as a direct compression, a wet granulation (with water or organic solvents) or a dry granulation process. Preferably, a granulation process is carried out in a fluid bed granulator.

Preferred is a process for preparing the composition according to the invention, wherein
(a) a solution of the active ingredient and the alkali metal additive in a solvent is sprayed on a carrier to obtain a granulate,
(b) optionally the granulate is mixed with other components,
(c) the granulate of (a) or (b) is compressed into tablets,
(d) optionally the tablets are provided with a coating, and
(e) the tablets of (c) or (d) are exposed to an atmosphere comprising 1 to 16 % by volume of oxygen.

Surprisingly, this way of processing the active ingredient results in a composition with an improved dissolution rate. It is also assumed that the applying of a solution on the carrier leads to an even distribution of the alkali metal additive around the active ingredient thereby contributing to the effective stabilizing of the active ingredient. This is only possible in view of the solubility of sodium hydroxide and potassium hydroxide in contrast to the virtually insoluble calcium carbonate and magnesium oxide used in the prior art compositions.

The solvent is preferably an alcohol and in particular methanol.

Moreover, it is preferred to use lactose or a lactose hydrate as carrier in step (a).

The other components of step (b) preferably include a mixture of croscarmelose sodium and crospovidone as disintegrant.

The present invention is in the following illustrated, but in no way limited by the following examples:

### Examples

### Example 1

| | Component | Amount per tablet/mg |
|---|---|---|
| 1 | Atorvastatin Ca | 41.44 |
| 2 | NaOH | 2.00 |
| 3 | Sodium Laurylsulfate | 8.00 |
| 4 | Hydroxypropyl cellulose | 8.00 |
| 5 | Methanol | about 0.3 |
| 6 | Lactose monohydrate | 222.56 |
| 7 | Microcrystalline cellulose | 70.00 |
| 8 | Croscarmelose sodium | 20.00 |
| 9 | Crospovidone | 20.00 |
| 10 | Talc | 4.00 |
| 11 | Magnesium stearate | 4.00 |
| | Total | 400 |

The sodium hydroxide was dissolved in methanol and atorvastatin calcium, sodium laurylsulfate as well as hydroxypropyl cellulose were added. The obtained granulating mixture was sprayed onto the lactose in a fluid bed granulator. The resulting granulate was dried and sieved and mixed with the components 7 to 11 and pressed into tablets using round, slightly biconvex punches.

The obtained tablet cores were further coated with Opadry II HP (3 % w/w of coating) and preferabyl a waxy surface active material based on lauryl macrogol glycerides having a melting point of about 50°C and a HLB value of about 13 as a second layer (2% w/w of caoting).

The tablets were packed in aluminium bags under reduced oxygen partial pressure and in air and stored at 40°C/75% relative humidity for 2, 4 and 8 weeks. The degradation products were determined by HPLC.

The oxygen content in blisters was measured using a mass spectrometer. A syringe needle was used for sampling of the blister atmosphere. The method involved removing a single foil blister from a pharmaceutical package containing atorvastatin tablets. On to the top of the blister was applied a small amount of silicon sealant to form an airtight seal. Once dry, a syringe needle was inserted into the sealant being careful not to pierce the blister. The needle was then evacuated to remove any air/oyxgen contamination before being inserted directly into the blister cavity. Headspace analysis was then performed using a mass spectrometer and the oxygen concentration was calculated from the measured O₂+/N₁₄N₁₅+ ion abundance ratio.

### Example 2

| | Component | Amount per tablet/mg |
|---|---|---|
| 1 | Atorvastatin Ca | 41.44 |
| 2 | NaOH | 2.00 |
| 3 | Sodium laurylsulfate | 8.00 |
| 4 | Hydroxypropyl cellulose | 15.00 |
| 5 | Methanol | About 0.3 |
| 6 | Lactose 200 mesh | 215.56 |
| 7 | Microcrystalline cellulose | 70.00 |
| 8 | Croscarmelose sodium | 20.00 |
| 9 | Crospovidone | 20.00 |
| 10 | Talc | 4.00 |
| 11 | Magnesium stearate | 4.00 |
| | Total | 400 |

Tablet cores were prepared as in example 1. These cores were further coated with Opadry II HP (3 % w/w of coating).

The tablets were packed in aluminium bags under reduced oxygen partial pressure and in air and stored at 40°C/75% relative humidity for 2, 4 and 8 weeks. Degradation products were determined by HPLC.

The oxygen content in blisters was measured using a mass spectrometer as explained above in example 1.

### Example 3 - Comparative composition

For comparative purposes a composition comprising the following ingredients but no NaOH was prepared.

| | | |
|---|---|---|
| | Component | Amount per tablet/mg |
| 1 | Atorvastatin Ca | 41.44 |
| 2 | Sodium Laurylsulfate | 8.00 |
| 3 | Hydroxypropyl cellulose | 16.00 |
| 4 | Methanol | About 0.3 |
| 5 | Lactose monohydrate | 146.00 |
| 6 | Microcrystalline cellulose | 82.56 |
| 7 | Amberlit IRP 80 | 58.00 |
| 8 | Croscarmelose sodium | 20.00 |
| 9 | Crospovidone | 20.00 |
| 10 | Talc | 4.00 |
| 11 | Magnesium stearate | 4.00 |
| | Total | 400 |

Atorvastatin calcium, sodium laurylsulfate and hydroxypropylcellulose were dissolved/suspended in methanol and the obtained granulating mixture was sprayed onto the lactose in a fluid bed granulator. The obtained granulate was dried and sieved, mixed with components 7-11 and pressed into tablets using round, slightly biconvex punches.

The obtained tablet cores were further coated with Opadry II HP (3 % w/w of coating).

The tablets were packed in aluminium bags under reduced oxygen partial pressure and in air and stored at 40°C/75% relative humidity for 2 and 4 weeks. The degradation products were again determined by HPLC.

### Example 4

| | Component | Amount per tablet/mg |
|---|---|---|
| 1 | Atorvastatin Ca | 41.44 |
| 2 | NaOH | 2.00 |
| 3 | Sodium Laurylsulfate | 8.00 |
| 4 | Hydroxypropyl cellulose | 8.00 |
| 5 | Methanol | about 0.3 |
| 6 | Lactose monohydrate | 222.56 |
| 7 | Microcrystalline cellulose | 70.00 |
| 8 | Croscarmelose sodium | 20.00 |
| 9 | Crospovidone | 20.00 |
| 10 | Silica, colloidal anhydrous | 2.00 |
| 11 | Sodium steryl fumarate | 6.00 |
| | Total | 400 |

The sodium hydroxide was dissolved in methanol and atorvastatin calcium, sodium laurylsulfate as well as hydroxypropyl cellulose were added. The obtained granulating mixture was sprayed onto the lactose in a fluid bed granulator. The resulting granulate was dried and sieved and mixed with the components 7 to 11 and pressed into tablets using round, slightly biconvex punches.

### Example 5 - Result of stability tests

### A. Composition according to the invention (example 1)

The results of the stability tests with the tablets according to Example 1 were as follows showing the amount of impurities other than the lactone of atorvastatin in air and an atmosphere containing about 4 Vol % of oxygen:

| | *Air* | *About 4 Vol* % *oxygen* |
|---|---|---|
| | Impurities | Impurities |
| To | 0.6 | 0.6 |
| After 2 weeks | 0.7 | 0.6 |
| After 4 weeks | 0.8 | 0.6 |
| After 8 weeks | 0.8 | 0.6 |

### B. Comparative composition (example 3)

The results of the stability tests with the tablets were of example 3 were as follows showing the amount of impurities other than the lactone of atorvastatin :

| | *Air* | *About* 4 *Vol* % *oxygen* |
|---|---|---|
| | Impurities | Impurities |
| To | 0.67 | 0.67 |
| After 2 weeks | 0.94 | 0.69 |
| After 4 weeks | 1.31 | 0.91 |

It can clearly be seen that the use of only a small amount of NaOH in combination with an atmosphere with a slightly reduced oxygen content provides a synergistic effect with regard to the prevention of undesired degradation product upon storage.

There was surprisingly no increase at all of the amount of the impurities to be detected even after 8 weeks.

## Claims

1. Pharmaceutical composition which comprises amorphous atorvastatin, a salt or ester thereof as active ingredient and an alkali metal additive selected from sodium hydroxide and potassium hydroxide and which is exposed to an atmosphere comprising 1 to 16 % by volume of oxygen.

2. Composition according to claim 1, which comprises amorphous atorvastatin calcium.

3. Composition according to claim 1 or 2, which comprises sodium hydroxide as alkali metal additive.

4. Composition according to any one of claims 1 to 3, wherein the atmosphere comprises 2 to 12 % by volume of oxygen.

5. Composition according to any one of claims 1 to 4, wherein the atmosphere comprises 4 to 10 % by volume of oxygen.

6. Composition according to any one of claims 1 to 5, which comprises 2 to 20, preferably 5 to 15 and most preferred 8 to 13 % by weight of the active ingredient.

7. Composition according to any one of claims 1 to 6, which comprises 0.01 to 5, preferably 0.1 to 3 and most preferred 0.2 to 1 % by weight of the alkali metal additive.

8. Composition according to any one of claims 1 to 7, which comprises at least one of croscarmelose sodium and crospovidone as disintegrant.

9. Composition according to claim 8, which comprises a mixture of corscarmelose sodium and crospovidone.

10. Composition according to claim 9, which comprises 5 to 15 and preferably 8 to 12 % by weight of the mixture.

11. Composition according to any one of claims 1 to 10, which is in form of a tablet and preferably a tablet having a coating.

12. Composition according to claim 11, wherein the coating comprises carboxymethyl cellulose or polyvinyl alcohol.

13. Composition according to claim 11 or 12, wherein the coating comprises a waxy material which is surface active.

14. Composition according to claim 13, wherein the waxy and surface active material has a melting point of about 33°C to about 64°C and in particular about 35°C to about 55°C.

15. Composition according to claim 13 or 14, wherein the waxy and surface active material has a HLB value of from about 1 to about 14, in particular from about 7 to about 14.

16. Composition according to any one of claims 13 to 15, wherein the waxy and surface active material comprises fatty acid esters of glycerol, PEG esters and/or free PEG (lauryl macrogol glycerides).

17. Composition according to any one of claims 1 to 16, which is present in a packaging.

18. Composition according to claim 17, wherein the packaging is a blister packaging or a bottle.

19. Process for preparing the composition according to any one of claims 1 to 18, wherein
(a) a solution of the active ingredient and the alkali metal additive in a solvent is sprayed on a carrier to obtain a granulate,
(b) optionally the granulate is mixed with other components,
(c) the granulate of (a) or (b) is compressed into tablets,
(d) optionally the tablets are provided with a coating, and
(e) the tablets of (c) or (d) are exposed to an atmosphere comprising 1 to 16 % by volume of oxygen.

20. Process according to claim 19, wherein the solvent is an alcohol, preferably methanol.

21. Process according to claims 19 or 20, wherein the carrier is lactose or a lactose hydrate.

22. Process according to any one of claims 19 to 21, wherein the other components include a mixture of croscarmelose sodium and crospovidone as disintegrant.
